# EUROPÄISCHE PATENTSCHRIFT

(11) **EP 2 515 998 B1**
(45) Veröffentlichungstag und Bekanntmachung des Hinweises auf die Patenterteilung: **25.02.2015**
(21) Anmeldenummer: 10805602.9
(22) Anmeldetag: 22.12.2010
(51) Int. Cl.: A61N 2/00, A61N 2/02, A61N 2/06

(54) **VERFAHREN ZUR KOSMETISCHEN HAUTGLÄTTUNG MITTELS MAGNETFELD**
METHOD OF COSMETIC SMOOTHING OF SKIN BY APPLYING MAGNETIC FIELD
PROCÉDÉ DE LISSAGE COSMÉTIQUE DE LA PEAU PAR LE CHAMP MAGNÉTIQUE

(30) Priorität: 23.12.2009 DE 102009060543
(43) Veröffentlichungstag der Anmeldung: 31.10.2012
(73) Patentinhaber: Muntermann, Axel, 35580 Wetzlar-Nauborn (DE)
(72) Erfinder: Muntermann, Axel, 35580 Wetzlar-Nauborn (DE)
(74) Vertreter: Schmidt, Oliver
(86) Internationale Anmeldenummer: PCT/EP2010/007855
(87) Internationale Veröffentlichungsnummer: WO 2011/076394

(56) Entgegenhaltungen:
- EP-A2- 1 787 678
- WO-A1-02/092167
- WO-A1-2005/075019
- WO-A2-2009/156117
- US-A1- 2003 069 464

## Beschreibung

### Gebiet der Erfindung

Die Erfindung betrifft ein Verfahren zur kosmetischen Hautglättung unter Verwendung von Kernspinresonanz.

### Hintergrund der Erfindung

Die Erfindung bezieht sich auf ein kosmetisches Verfahren zur Hautglättung, insbesondere bei Cellulite.

Bei Cellulite handelt es sich um eine Dellenbildung der Haut aufgrund einer Steppdecken-ähnlichen Unterteilung des Fettgewebes der Unterhaut. Es handelt sich mithin nicht um eine Krankheit, sondern um eine biologisch bedingte Veränderung des Bindegewebes. Auch die menschliche Haut als solche ist verschiedenen Alterserscheinungen wie Faltenbildung, schlaffe Haut durch dünner werdendes Bindegewebe usw. unterworfen. Obwohl es sich hierbei um normale Alterserscheinungen handelt, sind derartige Hautveränderungen für viele Menschen äußerst unerwünscht, und es gibt eine Vielzahl von kosmetischen, aber auch chirurgischen Ansätzen, die Haut zu glätten.

Bei Cellulite versucht man, insbesondere durch Lymphdrainage sowie durch Massagen die Durchblutung der Haut anzuregen. Als Ansatz, bei welchem technische Geräte verwendet werden, ist die Behandlung mittels Unterdruck in einer Vakuumröhre sowie die akustische Schallwellentherapie bekannt.

Diese bekannten Verfahren zur Hautglättung sind recht aufwändig und unangenehm. Weiter ist der erzielbare Behandlungserfolg umstritten.

### Aufgabe der Erfindung

Der Erfindung liegt die Aufgabe zugrunde, ein wirksames kosmetisches Verfahren zur Hautglättung, insbesondere bei Cellulite, bereit zu stellen.

Insbesondere soll mit der Erfindung ein Verfahren zur Hautglättung bereitgestellt werden, welches einfach durchzuführen und frei von schädlichen Nebenwirkungen ist.

### Zusammenfassung der Erfindung

Die Aufgabe der Erfindung wird durch ein Verfahren zur kosmetischen Hautglättung nach Anspruch 1 gelöst.

Bevorzugte Ausführungsformen und Weiterbildungen der Erfindung sind den Unteransprüchen zu entnehmen. Danach ist ein Verfahren zur kosmetischen Hautglättung vorgesehen, welches insbesondere bei schlaffer Haut und Cellulite sowie bei Falten, insbesondere im Gesicht, zur Anwendung kommt.

Gemäß des Verfahrens wird ein quasi-statisches Magnetfeld im Bereich des zu glättenden Hautareals und gleichzeitig ein senkrecht stehendes magnetisches Wechselfeld erzeugt.

Durch die Überlagerung vom statischen Feld mit dem magnetischen Wechselfeld kann Kernspinresonanz im zu behandelnden Hautareal erzielt werden.

Weiter wird die magnetische Feldstärke des quasi-statischen Magnetfeldes und/oder die Frequenz des magnetischen Wechselfeldes moduliert.

Durch die Modulation lässt sich auf recht einfache Weise sicherstellen, dass im gesamten zu behandelnden Hautareal zumindest zeitweise die Resonanzbedingung erreicht wird, wie im Folgenden noch detaillierter dargestellt wird.

Das zu behandelnde Hautareal wird einem im Wesentlichen statischen Magnetfeld ausgesetzt, welches zu einer Ausrichtung der Spins im zu behandelnden Gewebe führt. Über ein im Wesentlichen senkrecht zu dem statischen Feld stehendes magnetisches Wechselfeld wird das Umklappen von Spins herbeigeführt, also eine Kernspinresonanz erzeugt.

Vorzugsweise wird Kernspinresonanz wiederholt erzeugt, insbesondere über einen sogenannten schnellen adiabatischen Durchlauf (fast adiabatic passage), bei welchem die Amplitude des "statischen" Feldes moduliert wird, wodurch trotz Inhomogenitäten im magnetischen Feld eine Kernspinresonanz in einem großen Volumen erzeugt wird, so dass auch größere Hautareale, wie z.B. der gesamte Gesichtsbereich, auf einmal behandelt werden können.

Unter einem schnellen adiabatischen Durchlauf versteht man, dass die Frequenz des statischen Feldes derart langsam geändert wird, dass die Magnetisierung der Orientierung des fluktuierenden Feldes folgen kann, gleichzeitig aber schnell genug ist, dass die Magnetisierung nicht aufgrund von Relaxation verloren geht.

Es versteht sich, dass diese Darstellung auf einer klassischen Betrachtung des Kernspinresonanzeffektes beruht und dass dieser beschriebene Idealzustand nur als Hilfsmittel dient, den statistisch zu betrachtenden Effekt zu erklären.

Die Verwendung von Kernspinresonanzgeräten ist im übrigen in der Medizintechnik vor allem als bildgebendes Diagnoseverfahren bekannt. Derartige Diagnosegeräte arbeiten aber bei höheren Feldstärken, bei denen sich eine Verwendung im kosmetischen Bereich ohne ärztliche Mitwirkung nicht anbietet.

Ein quasi-statisches Magnetfeld im Sinne der Erfindung ist ein Magnetfeld, mit welchem die Spins bei klassischer Betrachtung ausgerichtet werden. Trotz der Bezeichnung als "quasi-statisches" Feld ist nicht ausgeschlossen, dass die Feldstärke dieses Feldes moduliert wird.

Weiter umfasst eine Vorrichtung zur Erzeugung von Kernspinresonanz eine Einrichtung zur Erzeugung eines magnetischen Wechselfeldes, welches vorzugsweise im Wesentlichen senkrecht zum quasi-statischen Magnetfeld steht.

Insbesondere zur Erzielung eines schnellen adiabatischen Durchlaufs ist die Feldstärke des quasi-statischen Magnetfeldes mit einer Modulationsfrequenz f_{M} modulierbar. Die Modulation erfolgt vorzugsweise linear, so dass sich ein Dreieckssignal ergibt. Andere Signalformen, bei welchen beispielsweise der Anstieg und/oder Fall der Feldstärke im Wesentlichen in der Mitte zwischen Minimum und Maximum verlangsamt, sind auch denkbar.

Alternativ kann statt oder neben der Feldstärke des quasi-statischen Magnetfeldes die Frequenz des magnetischen Wechselfeldes moduliert werden, da die Resonanzbedingung von der Feldstärke des statischen Magnetfeldes abhängt. Vorzugsweise wird aber die Feldstärke des quasi-statischen Magnetfeldes moduliert, da dies technisch einfacher realisierbar ist als eine Frequenzmodulation des magnetischen Wechselfeldes.

Bei einer Weiterbildung der Erfindung wird vor und/oder während des Erzeugens der Magnetfelder eine Creme appliziert, die insbesondere zumindest einen folgender Bestandteile aufweist: Wasser, Hyaluronsäure, Ubichinon, Kollagen und/oder Vitamin E. Die genannten Stoffe sind zur kosmetischen Hautbehandlung bekannt. Es ist insbesondere bekannt, dass sich durch Vitamin E, Hyaluronsäure und Ubichinon, auch unter der Bezeichnung Q11 bekannt, Glättungseffekte der Haut erzielen lassen.

Der Erfinder hat herausgefunden, dass durch die Erzeugung von Kernspinresonanz im zu behandelnden Hautareal ein synergetischer Effekt vorhanden ist, der ein wesentlich verbessertes Ergebnis nach sich zieht. Der Erfinder vermutet, dass dieser Effekt darauf zurückzuführen ist, dass die Kernspinresonanz die Aufnahmefähigkeit der Haut erheblich verbessert.

Der Erfinder hat des Weiteren herausgefunden, dass signifikante Behandlungserfolge vor allem bei recht geringen Feldstärken von weniger als 50 Gauß, bevorzugt von weniger als 30 Gauß und bei recht geringen Modulationsfrequenzen f_{M} von weniger als 100 Hz erreicht werden.

Insbesondere ist eine Modulationsfrequenz zwischen 1 und 50 Hz, bevorzugt von 5 Hz ± 3 Hz und besonders bevorzugt von 5 Hz ± 2 Hz geeignet, um einen Behandlungserfolg zu erzielen.

Auch die Frequenz des magnetischen Wechselfeldes ist recht gering und beträgt vorzugsweise weniger als 100 kHz und besonders bevorzugt weniger als 50 kHz.

Überraschenderweise führen gerade nicht starke Magnetfelder zum gewünschten Ergebnis, sondern bereits über geringe Feldstärken wird eine Hautglättung erreicht. Neben der überraschenden Erkenntnis, dass starke Felder gerade nicht zu verbesserten Erfolgen führen, wird durch diese Erkenntnis des Weiteren ermöglicht, ein Gerät bereitzustellen, welches aufgrund der schwachen erzeugten Felder ungefährlich ist und auch ohne geschultes Personal in Kosmetikstudios oder vom Benutzer selbst bedient werden kann.

Vorzugsweise weist die Vorrichtung, die bei Ausführung des Verfahrens Verwendung findet, eine Einrichtung auf, um eine Modulationsfrequenz f_{M} von weniger als 100 Hz einzustellen.

Der Erfinder hat herausgefunden, dass der Behandlungserfolg maßgeblich von der Modulationsfrequenz abhängt, wobei sich bei Modulationsfrequenzen von unter 100 Hz signifikant bessere Behandlungserfolge einstellen als bei höheren Frequenzen.

Vorzugsweise wird eine Modulationsfrequenz f_{M} zwischen 0,1 Hz und 100 Hz, besonders bevorzugt zwischen 2 Hz und 50 Hz ausgewählt.

Man war vorher davon ausgegangen, dass die Modulationsfrequenz alleine der Erzielung eines schnellen adiabatischen Durchlaufs im physikalischen Sinne dient und, sofern ein schneller adiabatischer Durchlauf erreicht wird, ein Behandlungserfolg nicht von der Modulationsfrequenz abhängt.

Bei einer Weiterbildung der Erfindung sind mittels der Einrichtung zur Einstellung einer Modulationsfrequenz eine Mehrzahl im Wesentlichen diskreter Frequenzen einstellbar, die zumindest 1 Hz, vorzugsweise 2 Hz, und besonders bevorzugt zumindest 5 Hz auseinander liegen.

Der Erfinder hat herausgefunden, dass in vielen Fällen unterschiedliche Modulationsfrequenzen optimal sind.

Über eine Einrichtung zur Einstellung der Modulationsfrequenz kann die für die jeweilige Indikation, beispielsweise Hauttyp und Faltenmuster, optimale Modulationsfrequenz auf einfache Weise ausgewählt werden.

Die Einrichtung zur Einstellung der Modulationsfrequenz kann dazu einen einfachen Wahlschalter umfassen, über den der Behandelnde die Indikation auswählt.

Alternativ oder in Kombination umfasst die Einrichtung zur Einstellung der Modulationsfrequenz einen Kartenleser, beispielsweise einen Chip- oder Magnetkartenleser. Auf der Karte kann das für die jeweilige Indikation optimale Frequenzmuster der Behandlungsablauf, die Behandlungszeit und/oder weitere relevante Parameter gespeichert sein.

Der Erfinder hat beispielsweise herausgefunden, dass eine Modulationsfrequenz von etwa 5 Hz zur Glättung der Haut, insbesondere bei Cellulite, die besten Ergebnisse erzielt wird.

Das magnetische Wechselfeld hat vorzugsweise eine Frequenz von weniger als 100 kHz, besonders bevorzugt von weniger als 50 kHz. Der Erfinder hat herausgefunden, dass bei Frequenzen von über 100 kHz der Behandlungserfolg signifikant abnimmt.

Vorzugsweise ist die Vorrichtung zur Erzeugung von Kernspinresonanz derart ausgebildet, dass zumindest eine der folgenden Modulationsfrequenzen auswählbar ist:
5 Hz ± 2 Hz
10 Hz ± 3 Hz
20 Hz ± 5 Hz
40 Hz ± 5 Hz.

Überraschenderweise nimmt bei höheren Frequenzen der Behandlungserfolg wiederum ab.

Bei einer Weiterbildung der Erfindung weist die Vorrichtung eine Einrichtung zur Einstellung einer Abfolge von mindestens zwei verschiedenen Modulationsfrequenzen auf.

Bei einer bevorzugten Ausführungsform der Erfindung ist die Einrichtung zur Erzeugung des quasi-statischen Magnetfeldes derart ausgebildet, dass eine Feldstärke von weniger als 50 Gauß, bevorzugt von weniger als 30 Gauß erreicht wird. Wie bereits zuvor dargestellt, hat sich gezeigt, dass niedrige Feldstärken und damit niedrige Frequenzen des magnetischen Wechselfeldes zu besseren Behandlungserfolgen führen.

Des Weiteren liegen die Feldstärken in einem so geringen Bereich, dass die erfindungsgemäße Vorrichtung auch ohne geschultes Fachpersonal betrieben werden kann.

Ähnliches gilt im Hinblick auf den von der Vorrichtung erzeugten Energieeintrag, welcher im zu behandelnden Gewebe weniger als 1 mW/l, vorzugsweise weniger als 100 µW/l und besonders bevorzugt weniger als 10 µW/l beträgt. Es hat sich gezeigt, dass die Behandlungserfolge gerade nicht von einem hohen Energieeintrag im zu behandelnden Gewebe abhängen. Die durch die Kernspinresonanz hervorgerufenen derart niedrigen Energieeinträge stellen zudem sicher, dass durch die kosmetische Behandlung weder eine Gefahr für den Kunden noch für das Personal besteht.

Sofern die Feldstärke des quasi-statischen Magnetfeldes moduliert wird, wird vorzugsweise mit einem Betrag von zumindest 5 %, bevorzugt zumindest 10 % und besonders bevorzugt zumindest 15 % der Mindestfeldstärke des quasi-statischen Magnetfeldes moduliert. Das quasi-statische Magnetfeld besteht also aus einem Sockelbetrag, welcher um einen Modulationsbetrag erhöht wird. Eine Modulation von über 5 % des Sockelbetrages reicht in der Regel aus, um nicht nur Inhomogenitäten des von der Vorrichtung selbst erzeugten Feldes innerhalb des zu behandelnden Gewebes auszugleichen, sondern auch, um Inhomogenitäten aufgrund externer Magnetfelder, welche sich mit dem Magnetfeld der Vorrichtung überlagern, zu kompensieren. Dies gilt im Besonderen für das Erdmagnetfeld, wobei bei einer bevorzugten Ausführungsform der Erfindung die Feldstärke des quasi-statischen Magnetfeldes um das Zweifache, bevorzugt um das zumindest Dreifache und besonders bevorzugt um das zumindest das Sechsfache der magnetischen Feldstärke des Erdmagnetfeldes moduliert wird.

Das gleiche Prinzip ist bei einer alternativen Ausführungsform der Erfindung, bei welcher die Frequenz des magnetischen Wechselfeldes modulierbar ist, ebenfalls anwendbar. Auch hier kann das magnetische Wechselfeld um einen Betrag von zumindest 5 %, vorzugsweise zumindest 10 % und besonders bevorzugt zumindest 15 % der Mindestfrequenz des magnetischen Wechselfeldes moduliert werden. Um das Erdmagnetfeld auszugleichen, kann die Modulation des magnetischen Wechselfeldes zumindest 2 Hz, vorzugsweise zumindest 4 Hz und besonders bevorzugt zumindest 6 Hz betragen.

Bei einer bevorzugten Ausführungsform der Erfindung ist die Vorrichtung derart ausgebildet, dass eine Modulationsfrequenz ausgewählt ist, die zu einer Magnetisierung von zumindest 60 %, vorzugsweise von zumindest 70 % der maximalen Magnetisierung im zu behandelnden Gewebe führt. Unter der maximalen Magnetisierung wird die Magnetisierung verstanden, welche bei der jeweils angelegten Feldstärke des quasi-statischen Feldes maximal im zu behandelnden Gewebe erreicht werden kann. Beim idealen schnellen adiabatischen Durchlauf nähert sich die Magnetisierung im zu behandelnden Gewebe dem Wert der theoretisch möglichen maximalen Magnetisierung. Der Erfinder hat herausgefunden, dass eine Mindestmagnetisierung von etwa 60 % vorhanden sein sollte, aber auch ausreichend ist, um Erfolge bei der Glättung von Haut zu erreichen.

Bei einer Weiterbildung der Erfindung ist die Frequenz des magnetischen Wechselfeldes über eine Bewegung von Platten eines Kondensators gegeneinander modulierbar. Der Erfinder hat herausgefunden, dass dies ein besonders einfaches Verfahren ist, eine stetige Veränderung der Frequenz des magnetischen Wechselfeldes zu erreichen. In den bevorzugten Frequenzbereichen der Modulationsfrequenz ist insbesondere eine motorische Ansteuerung der Platten des Kondensators möglich.

Alternativ kann die Frequenz des magnetischen Wechselfeldes auch über die Zuschaltung von Kondensatoren moduliert werden. Ein derartiger Aufbau ist schaltungstechnisch allerdings wesentlich aufwändiger, insbesondere wenn die Vorrichtung mit hohen Spannungen betrieben wird.

Weiter betrifft die Erfindung ein Verfahren Hautstraffung unter Verwendung von Kernspinresonanz, bei welchem über ein quasi-statisches Magnetfeld sowie ein das quasi-statische Magnetfeld überlagerndes magnetisches Wechselfeld Kernspinresonanz im zu behandelnden Hautgewebe erzeugt werden.

Dabei wird die magnetische Feldstärke des quasi-statischen Magnetfeldes und/oder die Frequenz des magnetischen Wechselfeldes periodisch mit einer Modulationsfrequenz f_{M} moduliert, wobei zur Behandlung eine Modulationsfrequenz f_{M} von weniger als 100 Hz verwendet wird.

Wie zuvor dargestellt, führen niedrige Modulationsfrequenzen von weniger als 100 Hz, insbesondere Modulationsfrequenzen zwischen 1 Hz und 50 Hz zu überraschenden Behandlungserfolgen.

Vorzugsweise beträgt die Behandlungszeit bei einer Sitzung zwischen einer und 150 Minuten, besonders bevorzugt zwischen 3 und 60 Minuten.

Die Erfindung betrifft des Weiteren eine Vorrichtung zur kosmetischen Hautbehandlung, welche eine Einrichtung zur Erzeugung eines quasi-statischen Magnetfeldes sowie eine Einrichtung zur Erzeugung eines magnetischen Wechselfeldes umfasst und wobei die Feldstärke des statischen Feldes und/oder die Frequenz des magnetischen Wechselfeldes mit einer Modulationsfrequenz f_{M} modulierbar ist.

Die Vorrichtung umfasst eine Grundfläche und zwei sich von den Seiten der Grundfläche aus erstreckende abgewinkelte Seitenteile, wobei in der Grundfläche die Einrichtung zur Erzeugung des magnetischen Wechselfeldes, insbesondere in Form einer Spule angeordnet ist und wobei in den Seitenteilen die Vorrichtung zur Erzeugung des quasi-statischen Magnetfeldes angeordnet ist.

Die Vorrichtung zur Erzeugung des quasi-statischen Magnetfeldes ist derart ausgebildet, dass sich die Feldlinien zumindest abschnittsweise von einem Seitenteil zum anderen Seitenteil erstrecken.

Dies gelingt insbesondere durch in den Seitenteilen angeordnete Spulen.

Durch die Erfindung wird gegenüber herkömmlichen bildgebenden Kernspinresonanzgeräten ermöglicht, ein Behandlungsgerät bereitzustellen, welches gegenüber der Grundfläche offen ist und welches eine sehr große Behandlungszone aufweist, die sich zwischen den beiden Seitenteilen erstreckt und innerhalb der in einem großen Volumen Kernspinresonanzen erzeugbar sind.

Eine derartige Vorrichtung eignet sich besonders für die kosmetische Verwendung, da der Patient nicht in eine Röhre geschoben werden muss.

Vielmehr kann die Einrichtung zur Erzeugung von Kernspinresonanz als Modul ausgebildet sein, welches beispielsweise Teil eines verstellbaren Behandlungsstuhles zur Gesichtsbehandlung ist oder welches beispielsweise in einer etwas kleineren Ausführungsform mittels eines Textilbandes direkt am Kunden befestigt werden, beispielsweise am Arm oder Bein.

Die Erfindung betrifft des Weiteren die Verwendung von Kernspinresonanz zur kosmetischen Hautglättung, insbesondere bei Cellulite.

### Kurzbeschreibung der Zeichnungen

Die Erfindung soll im Folgenden Bezug nehmend auf die Zeichnungen Fig. 1 bis Fig. 5, welche schematisch dargestellte Ausführungsbeispiele der Erfindung zeigen, näher erläutert werden.
- Fig. 1: zeigt schematisch die Feldstärken der beiden magnetischen Felder bei einer Ausführungsform, bei welcher das quasi-statische Magnetfeld moduliert wird,
- Fig. 2: zeigt schematisch die wesentlichen Bestandteile einer Vorrichtung zur Erzeugung von Kernspinresonanz,
- Fig. 3: zeigt schematisch einen Aufbau mit einer Vorrichtung zur Erzeugung von Kernspinresonanzresonanz,
- Fig. 4 und 5: zeigen schematisch eine alternative Vorrichtung zur Erzeugung von Kernspinresonanzresonanz,
- Fig. 6: zeigt die in Fig. 4 und 5 dargestellte Vorrichtung zur Erzeugung von Kernspinresonanz auf einem Behandlungsstuhl montiert,
- Fig. 7: zeigt den Verlauf der Magnetisierung im zu behandelnden Gewebe,
- Fig. 8: zeigt den Verlauf des statischen Magnetfeldes sowie der Frequenz des magnetischen Wechselfeld bei einer alternativen Ausführungsform der Erfindung, bei der die Frequenz des magnetischen Wechselfeld moduliert wird.
- Fig. 9: zeigt eine weitere alternative Vorrichtung zur Erzeugung von Kernspinresonanz.

### Detaillierte Beschreibung der Zeichnungen

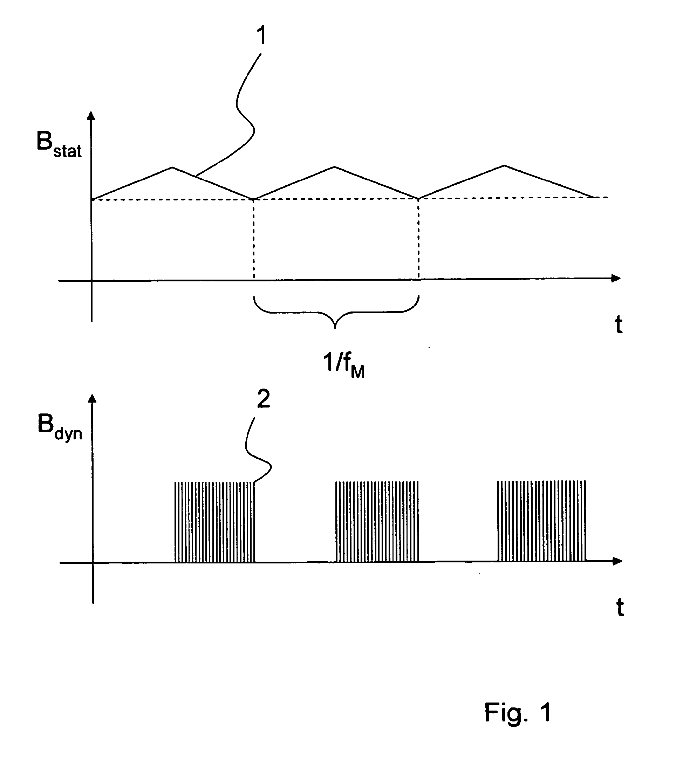

In Fig. 1 ist im oberen Graphen das statische Magnetfeld gegen die Zeit aufgetragen und im unteren Graphen das magnetische Wechselfeld gegen die Zeit aufgetragen.

Zu erkennen ist, dass die Feldstärke des quasi-statischen Magnetfeldes 1 einen Sockelbetrag umfasst, welcher mit der gestrichelten Linie gekennzeichnet ist. Über den Sockelbetrag hinaus wird die magnetische Feldstärke des quasi-statischen Magnetfeldes 1 mit einem Dreieckssignal moduliert, wodurch sich ein linearer Anstieg und Abfall des Feldes ergibt. Ein kompletter Durchlauf, das heißt ein Ansteigen und wieder Abfallen auf die Amplitude des Sockelbetrages entspricht 1 f_{M}.

Zur Erzielung eines schnellen adiabatischen Durchlaufs wird jeweils während des Abfallens des statischen Magnetfeldes ein magnetisches Wechselfeld zugeschaltet. Die Feldstärke des magnetischen Wechselfeldes 2, welches in diesem Ausführungsbeispiel sinusförmig (nicht dargestellt) verläuft, ändert sich im Wesentlichen nicht. Aufgrund des An- und Abschaltens liegt vielmehr im Wesentlichen ein Rechtecksignal vor. Andere Signalverläufe sind aber auch denkbar.

Während des Abfalls der Feldstärke des quasi-statischen Feldes 1 wird im zu behandelnden Gewebe eine Kernspinresonanz erzeugt.

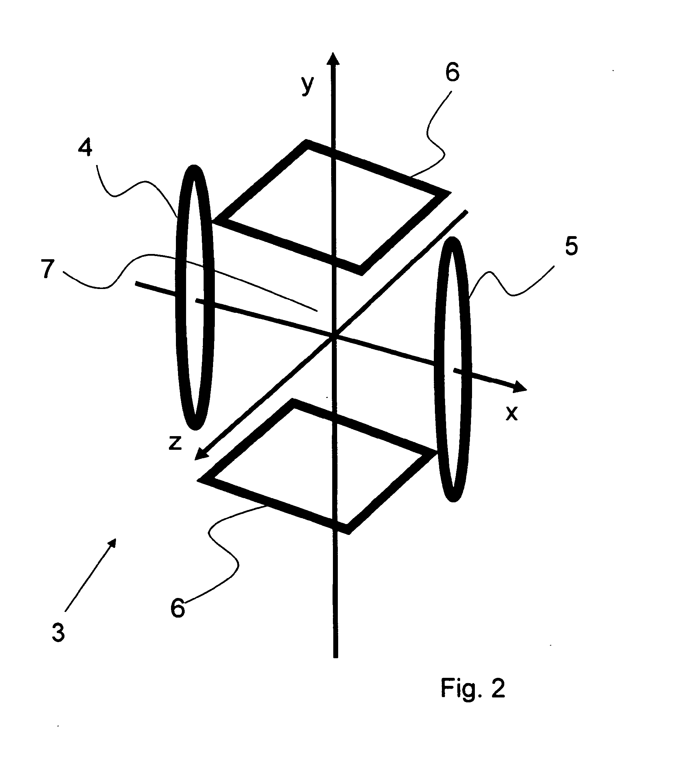

Bezug nehmend auf Fig. 2 sollen schematisch die wesentlichen Bestandteile einer ersten Vorrichtung zur Erzeugung von Kernspinresonanz 3 erläutert werden.

Die Vorrichtung umfasst eine Einrichtung zur Erzeugung eines quasi-statischen Feldes 6, welches in diesem Ausführungsbeispiel aus Permanentmagneten und einer Spule (nicht dargestellt) besteht. Der Permanentmagnet kann dabei das Grundfeld erzeugen, wohingegen die Spule die Modulation der Feldstärke übernimmt.

Über die Vorrichtung zur Erzeugung des quasi-statischen Feldes 6 wird in Richtung der y-Achse ein möglichst homogenes Feld innerhalb der Behandlungszone 7 erzeugt.

Weiter umfasst die Vorrichtung zur kosmetischen Hautglättung 3 die Spulen 4 und 5, mit welchen ein magnetisches Wechselfeld in Richtung der x-Achse erzeugt werden kann.

Das magnetische Wechselfeld überlagert somit das quasi-statische Magnetfeld und steht senkrecht zu diesem, wodurch Kernspinresonanz im zu behandelnden Gewebe erzeugt werden kann.

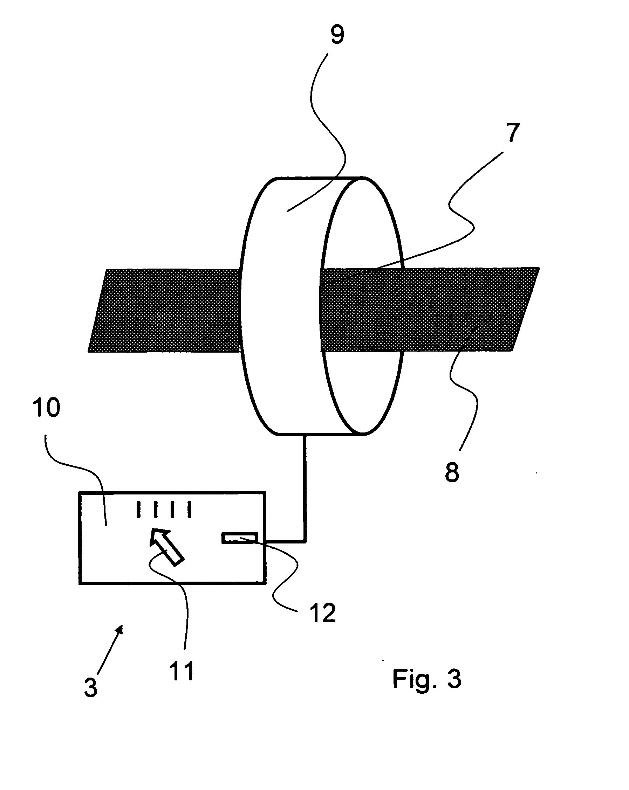

Fig. 3 zeigt schematisch eine Vorrichtung zur Erzeugung von Kernspinresonanz 3. Die Vorrichtung umfasst eine Ringröhre 9 mit den in Fig. 2 dargestellten Spulen.

Die Vorrichtung zur Erzeugung des quasi-statischen Felds ist in diesem Ausführungsbeispiel nicht explizit dargestellt.

Innerhalb der Ringröhre 9 ist die Behandlungszone 7 angeordnet. Während der Behandlung ruht der Kunde (nicht dargestellt) auf einer Liege 8, welche durch die Ringröhre 9 verläuft.

Die Vorrichtung umfasst des Weiteren eine Steuereinrichtung 10, mit welcher die in der Ringröhre befindlichen Spulen (nicht dargestellt) angesteuert werden. Die Steuereinrichtung 10 umfasst zum einen Wahlschalter 11 zur einfachen Auswahl verschiedener Modulationsfrequenzen.

Weiter umfasst in diesem Ausführungsbeispiel die Steuereinrichtung 10 einen Kartenleser 12. Über den Kartenleser 12 können auf einer Chip- oder Magnetkarte gespeicherte Behandlungsfrequenzen und andere relevante Parameter ausgelesen werden und somit ebenfalls auf einfache Art und Weise vom Benutzer ausgewählt werden.

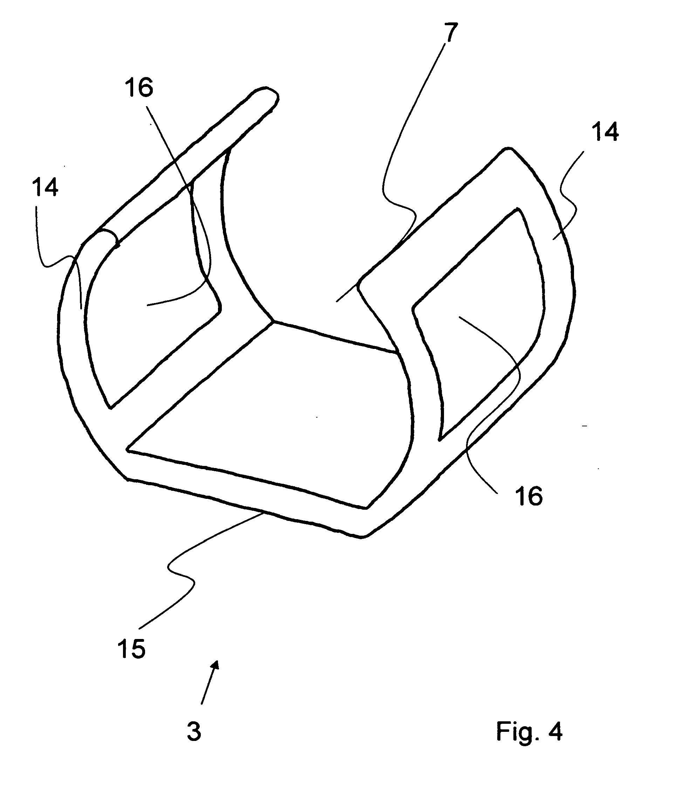

In Fig. 4 ist schematisch eine Vorrichtung zur Erzeugung von Kernspinresonanz 3 dargestellt, wie sie in dieser Ausführungsform besonders für kosmetische Zwecke geeignet ist.

Die Vorrichtung 3 umfasst eine Grundfläche 15, welche beispielsweise dazu dient, dass der Kunde seinen Kopf anlehnen kann.

Von der Grundfläche 15 aus erstrecken sich die beiden Seitenteile 14.

Die Seitenteile 14 sind in der Vorderansicht gebogen ausgebildet und haben einen in etwa kreissegmentförmigen beziehungsweise U-förmigen Querschnitt.

Weiter bilden die beiden Seitenteile 14 einen Rahmen und haben somit jeweils eine Aussparung 16.

Gegenüber der Grundfläche 15 ist die Vorrichtung 3 offen, was eine wesentlich leichtere Zugänglichkeit der zwischen Seitenteilen 14 und der Grundfläche 15 liegenden Behandlungszone 7 ermöglicht.

In der Grundfläche 15 befindet sich eine erste Einrichtung zur Erzeugung eines magnetischen Wechselfeldes (nicht dargestellt).

Die Vorrichtung zur Erzeugung von Kernspinresonanzen ist von Ihrer Dimensionierung her bei einer Ausführungsform auf die Behandlung der Gesichtshaut des Kunden ausgelegt.

Es ist aber auch vorgesehen, kleiner ausgebildete Vorrichtungen, beispielweise zur Behandlung der Haut an Armen und Beinen zu verwenden. Dabei kann die hier dargestellte Vorrichtung beispielweise mittel Bändern, insbesondere Klettbändern am Arm oder Bein des Kunden fixiert werden. Aber auch die Befestigung an einem Behandlungsstuhl ist denkbar.

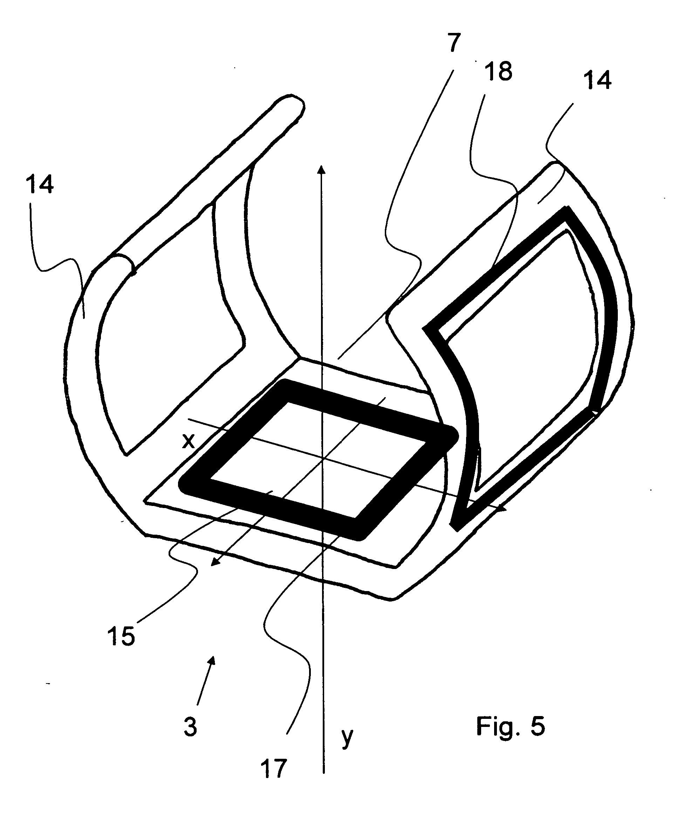

Fig. 5 zeigt die Ausführungsform einer Vorrichtung zur Erzeugung von Kernspinresonanzen gemäß Fig. 4, wobei in dieser Ausführungsform schematisch eine in der Grundfläche 15 integrierte Spule 17 eingezeichnet ist, welche in diesem Ausführungsbeispiel eine Einrichtung zur Erzeugung eines magnetischen Wechselfeldes bildet.

In den Seitenteilen 14 befindet sich jeweils eine Spule 11, über die ein Magnetfeld erzeugbar ist. Die Spule im linken Seitenteil ist nicht dargestellt, es ist aber ersichtlich, dass sich über die Spulen in den Seitenteilen 14 ein Magnetfeld erzeugen lässt, welches in der Behandlungszone 7 im Wesentlichen senkrecht zu dem von der Spule 17 erzeugten Magnetfeld steht.

Die Spulen können beispielsweise über ein netzunabhängiges Steuergerät, insbesondere mit einem Akkumulator versorgt werden (nicht dargestellt).

So kann innerhalb der Behandlungszone 7 Kernspinresonanz in dem zu behandelnden Hautareal(nicht dargestellt) erzeugt werden.

Über die in der Grundfläche 15 integrierte Spule 17 wird ein erstes Magnetfeld erzeugt, dessen Feldlinien in der Behandlungszone im Wesentlichen entlang der y-Achse verlaufen.

Über die Spulen in den Seitenteilen 14 wird ein dazu senkrechtes Feld erzeugt, dessen Feldlinien im Wesentlichen entlang der x-Achse verlaufen. Die Spulen in den Seitenteilen 14 bilden also eine zweite Einrichtung zur Erzeugung eines im Wesentlichen statischen magnetischen Feldes. Das erzeugte Feld ist aufgrund der Anordnung, die einer Helmholtzspule nahekommt, sehr homogen.

Es bietet sich daher an, zur Erzeugung von Kernspinresonanz im zu behandelnden Gewebe über die in den Seitenteilen 14 angeordneten Spulen ein im Wesentlichen statisches Magnetfeld zu erzeugen und über die Spule 17 ein senkrecht dazu stehendes Wechselfeld zu erzeugen, welches zum Umklappen der Spins führt.

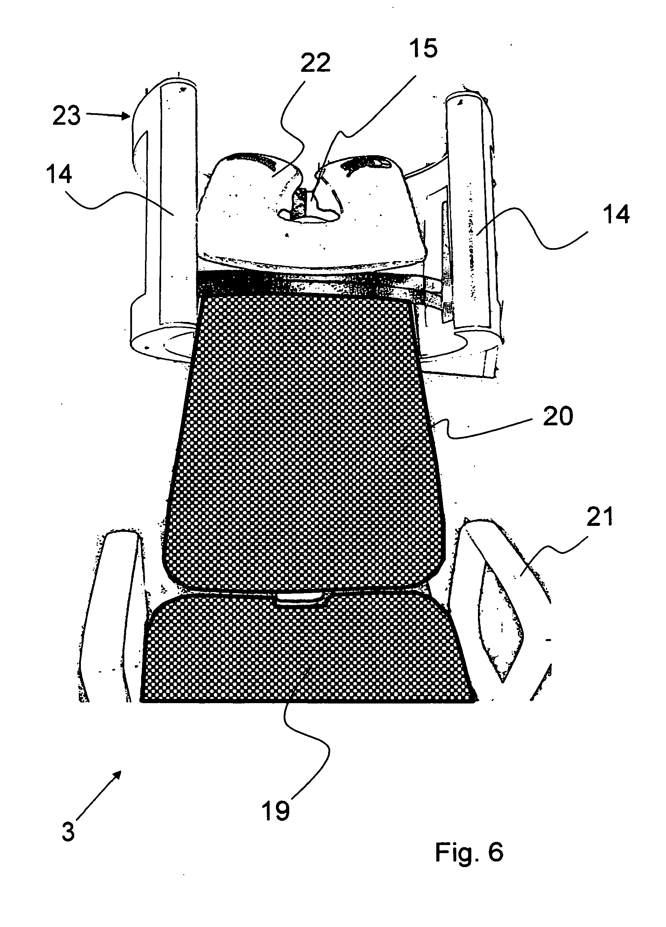

In Fig. 6 ist schematisch eine weitere Ausführungsform der Erfindung dargestellt, bei welcher die Vorrichtung zur Erzeugung von Kernspinresonanzen 3 eine Sitzfläche 19 aufweist, auf welche sich der Kunde setzen kann. Zur Steigerung der Bequemlichkeit umfasst die Vorrichtung noch eine Rückenlehne 20, eine Armlehne 21 sowie ein Nackenkissen 22.

Ein Modul 23, welches Grundfläche 15 und Seitenteile 14 umfasst ist im Wesentlichen vertikal angeordnet.

Vorzugsweise ist das Modul 23 zumindest in der Höhe verstellbar, um die Vorrichtung auf unterschiedliche Größen anpassen zu können oder um unterschiedliche Hautpartien zu behandeln.

Vorzugsweise ist der Sitzfläche 19 und Rückenlehne 20 aufweisende Behandlungsstuhl auch mit einer verstellbaren Beinstütze (nicht dargestellt) versehen und ist auch selbst vielfältig verstellbar ausgebildet, insbesondere höhenverstellbar, mit verstellbarer Rückenlehne und verstellbaren Armlehnen.

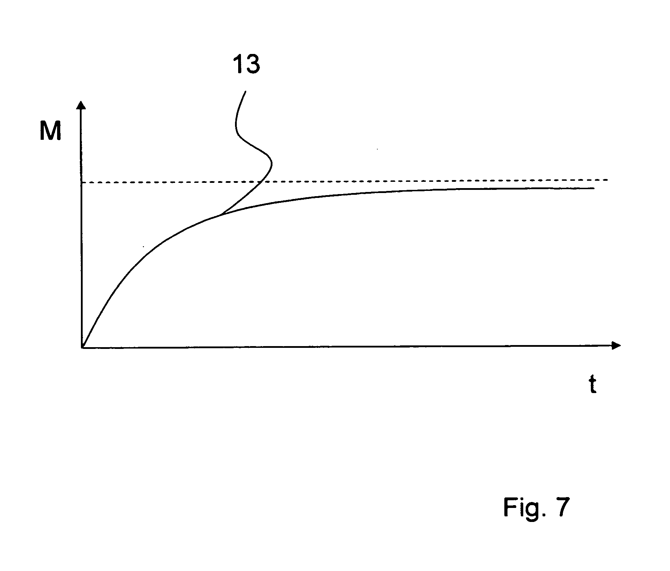

Bezug nehmend auf Fig. 7 soll schematisch die Magnetisierung im zu behandelnden Hautareal 13 erläutert werden. Die Geschwindigkeit, mit der die Spins mit den Nachbarn reagieren, ist gitterabhängig. Beim schnellen adiabatischen Durchlauf kann eine maximale Magnetisierung des zu behandelnden Gewebes erreicht werden, welche in diesem Fall mit einer gestrichelten Linie symbolisiert ist. In der Realität kann dieser Maximalwert nicht ganz erreicht werden, sondern die relative Magnetisierung des Gewebes nähert sich dem Maximalwert. Unter anderem mit Änderung der Modulationsfrequenz kommt die Magnetisierung diesem Maximalwert mehr oder weniger nahe. Der Erfinder hat herausgefunden, dass etwa 60 % Magnetisierung ausreichend ist, um signifikante Glättungserfolge zu erzielen.

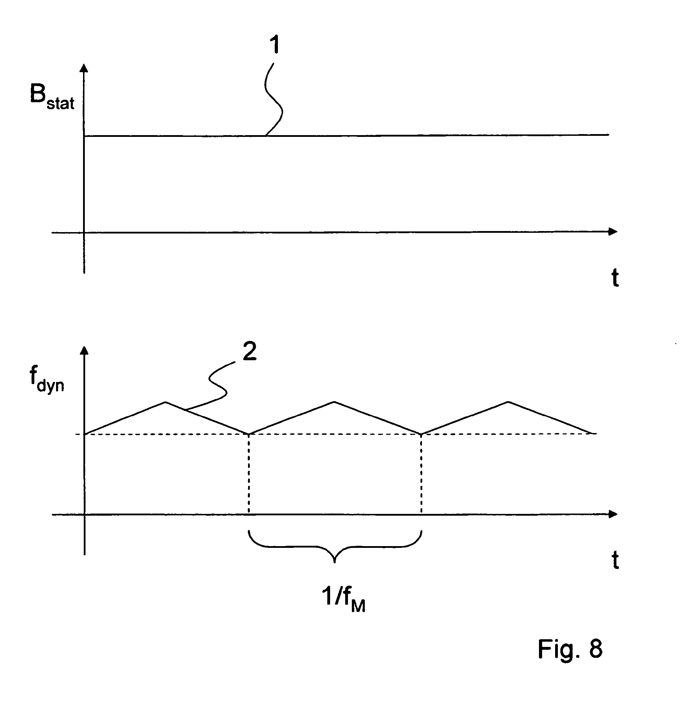

Bezug nehmend auf Fig. 8 soll eine alternative Ausführungsform der Erfindung erläutert werden, bei welcher die Frequenz des magnetischen Wechselfeldes moduliert wird.

Die Feldstärke dargestellten statischen Magnetfeldes 1 ist in diesem Ausführungsbeispiel konstant.

Unten dargestellt ist die Frequenz des magnetischen Wechselfeldes 2, welche als Dreieckssignal moduliert wird. Auch hier entspricht ein kompletter Durchlauf 1 f_{M}. Durch die Modulation des magnetischen Wechselfeldes lässt sich physikalisch das gleiche erreichen, wie durch eine Modulation des quasi-statischen Feldes. Es wird durch die Modulation der Frequenz des Wechselfeldes ein schneller adiabatischer Durchlauf im zu behandelnden Areal ermöglicht.

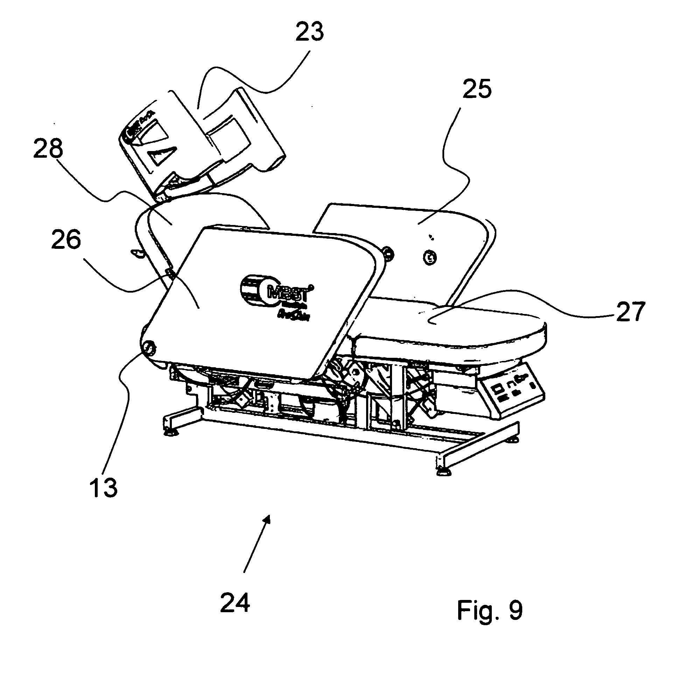

Fig. 9 zeigt eine weitere Ausführungsform einer Vorrichtung zur Erzeugung von Kernspinresonanz 24.

Die Vorrichtung zur Erzeugung von Kernspinresonanz 24 umfasst in diesem Ausführungsbeispiel eine motorisch verstellbare Liege 27 mit einer Rückenlehne 28 in welcher der Kunde nicht nur liegen, sondern auch aufrecht oder halbaufrecht sitzen kann.

Diese Vorrichtung zur Erzeugung von Kernspinresonanz 24 umfasst zwei Behandlungszonen.

Hierzu sind zwei sich neben der Liege 27 erstreckende Seitenteile 25, 26 vorgesehen. In den Seitenteilen ist jeweils eine Spule (nicht dargestellt) integriert. Mittels der in den Seitenteilen 25, 26 integrierten Spulen kann ein im Wesentlichen statisches Magnetfeld erzeugt werden, welches sich von einem Seitenteil zum anderen Seitenteil erstreckt. Unterhalb der Liege 27 befindet sich eine Spule (nicht dargestellt) zur Erzeugung eines magnetischen Wechselfeldes, welches zwischen den Seitenteilen 25 und 26 im Wesentlichen senkrecht zum statischen Magnetfeld steht. Zwischen den Seitenteilen 25 und 26 befindet sich somit eine erste Behandlungszone. Zum besseren Einstieg ist zumindest eines der Seitenteile 26 an einem Drehgelenk 13 angeordnet und kann in Richtung der Lehne 28 zur Seite hin weggeschwenkt werden.

Im Kopfbereich ist eine weitere, als Modul 23 ausgebildete Vorrichtung zur Erzeugung von magnetischen Feldern angeordnet. Dieses Modul 23 dient der Behandlung der Gesichtshaut. Die Spulenkonfiguration entspricht der im Zusammenhang mit Fig. 6 beschriebenen Spulenkonfiguration.

Mit der in Fig. 9 dargestellten Vorrichtung zur Erzeugung von Kernspinresonanz 24 kann somit die Haut des Kunden in zwei Behandlungszonen behandelt werden. Die Behandlung kann dabei unabhängig voneinander erfolgen, insbesondere kann das mit dem Modul 23 erzeugte statische Feld mit einer anderen Frequenz gesweept werden.

Es versteht sich, dass die Erfindung nicht auf eine Kombination vorstehend beschriebener Merkmale beschränkt ist, sondern dass der Fachmann sämtliche Merkmale, soweit dies sinnvoll ist, kombinieren wird.

### Bezugszeichenliste

- 1: Feldstärke des quasi-statischen Magnetfeldes
- 2: Feldstärke des magnetischen Wechselfeldes
- 3: Vorrichtung zur Erzeugung von Kernspinresonanz
- 4: Spule
- 5: Spule
- 6: Vorrichtung zur Erzeugung eines quasi-statischen Feldes
- 7: Behandlungszone
- 8: Liege
- 9: Ringröhre
- 10: Steuereinrichtung
- 11: Wahlschalter
- 12: Kartenleser
- 13: relative Magnetisierung des Hautareals
- 14: Seitenteil
- 15: Grundfläche
- 16: Aussparung
- 17: Spule
- 18: Spule
- 19: Sitzfläche
- 20: Rückenlehne
- 21: Armlehne
- 22: Nackenkissen
- 23: Modul
- 24: Vorrichtung zur Erzeugung von Kernspinresonanz
- 25: Seitenteil
- 26: Seitenteil
- 27: Liege
- 28: Rückenlehne

## Patentansprüche

1. Verfahren zur kosmetischen Hautglättung, umfassend folgende Schritte:
- Erzeugen eines quasi-statischen Magnetfeldes im Bereich des zu glättenden Hautareals,
- gleichzeitiges Erzeugen eines magnetisches Wechselfeldes im Bereich des zu glättenden Hautareals, welches zumindest abschnittsweise im Wesentlichen senkrecht zu dem quasi-statischen Magnetfeld steht,
- Modulieren der magnetischen Feldstärke des quasi-statischen Magnetfeldes und/oder der Frequenz des magnetischen Wechselfeldes,
- wobei während des Erzeugens der Magnetfelder eine Creme appliziert wird, die zumindest einen folgender Bestandteile aufweist:
- Wasser,
- Hyaluronsäure,
- Ubichinon,
- Kollagen,
- Vitamin E,

2. Verfahren zur kosmetischen Hautglättung nach dem vorstehenden Anspruch, **dadurch gekennzeichnet, dass** das Verfahren bei Cellulite eingesetzt wird.

3. Verfahren zur kosmetischen Hautglättung nach dem vorstehenden Anspruch, **dadurch gekennzeichnet, dass** die magnetische Feldstärke des quasi-statischen Magnetfeldes und/oder die Frequenz des magnetischen Wechselfeldes mit einer Modulationsfrequenz f_{M} moduliert wird, wobei die Modulationsfrequenz f_{M} weniger als 100 Hz oder zwischen 0,1 Hz und 100 Hz, vorzugsweise zwischen 1 Hz und 50 Hz beträgt.

4. Verfahren zur kosmetischen Hautglättung nach einem der vorstehenden Ansprüche, **dadurch gekennzeichnet, dass** während der Behandlung zumindest zwei verschiedene Modulationsfrequenzen verwendet werden.

5. Verfahren zur kosmetischen Hautglättung nach einem der vorstehenden Ansprüche, **dadurch gekennzeichnet, dass** der im zu behandelnden Gewebe erzeugte Energieeintrag auf weniger als 1 mW/l, vorzugsweise weniger als 100 *µ*W/l, besonders bevorzugt weniger als 10 *µ*W/l eingestellt wird.

6. Verfahren zur kosmetischen Hautglättung nach einem der vorstehenden Ansprüche, **dadurch gekennzeichnet, dass** durch die Modulation der Feldstärke des quasi-statischen Magnetfeldes und/oder der Frequenz des magnetischen Wechselfeldes ein schneller adiabatischer Durchlauf im zu behandelnden Gewebe erzeugt wird.

7. Verfahren zur kosmetischen Hautglättung nach einem der vorstehenden Ansprüche, **dadurch gekennzeichnet, dass** die Modulation der Feldstärke des quasi-statischen Magnetfeldes zumindest 5 % der Amplitude der Mindestfeldstärke des quasi-statischen Magnetfeldes beträgt.

8. Verfahren zur kosmetischen Hautglättung nach einem der vorstehenden Ansprüche, **dadurch gekennzeichnet, dass** die Modulation der Frequenz des magnetischen Wechselfeldes zumindest 5 % der Mindestfrequenz des magnetischen Wechselfeldes beträgt.

9. Verfahren zur kosmetischen Hautglättung nach einem der vorstehenden Ansprüche, **dadurch gekennzeichnet, dass** die Modulation der Feldstärke des quasi-statischen Magnetfeldes zumindest das Zweifache der magnetischen Feldstärke des Erdmagnetfeldes beträgt.

10. Verfahren zur kosmetischen Hautglättung nach einem der vorstehenden Ansprüche, **dadurch gekennzeichnet, dass** die Behandlungszeit zwischen 1 und 150 min beträgt.

11. Verfahren zur kosmetischen Hautglättung nach einem der vorstehenden Ansprüche, wobei Feldstärke des quasi-statischen Magnetfeldes auf weniger als 50 Gauß eingestellt wird.

12. Verfahren zur kosmetischen Hautglättung nach einem der vorstehenden Ansprüche, wobei die Frequenz des magnetischen Wechselfeldes weniger als 100 kHz beträgt.

13. Verfahren zur kosmetischen Hautglättung nach einem der vorstehenden Ansprüche, wobei durch die Modulation der Feldstärke des quasi-statischen Magnetfeldes und/oder der Frequenz des magnetischen Wechselfeldes ein schneller adiabatischer Durchlauf im zu behandelnden Gewebe erzeugt wird, wobei die Modulationsfrequenz derart ausgewählt ist, dass die Magnetisierung zumindest 60 % der maximalen Magnetisierung im zu behandelnden Gewebe beträgt.

## Claims

1. A method of cosmetic skin smoothing, comprising the steps of:
- generating a quasi-static magnetic field in the region of the skin area to be smoothened;
- simultaneously generating an alternating magnetic field in the region of the skin area to be smoothened, which is substantially perpendicular to said quasi-static magnetic field, at least in portions thereof;
- modulating the magnetic field strength of the quasi-static magnetic field and/or the frequency of the alternating magnetic field;
- wherein a cream is applied during the generation of the magnetic fields, which cream in particular comprises at least one of the following components:
- water,
- hyaluronic acid,
- ubiquinone,
- collagen,
- vitamin E.

2. The method for cosmetic skin smoothing according to the preceding claim, **characterized in that** the method is used for cellulite.

3. The method for cosmetic skin smoothing according to the preceding claim, **characterized in that** the magnetic field strength of the quasi-static magnetic field and/or the frequency of the alternating magnetic field is modulated with a modulation frequency f_{M}, wherein the modulation frequency f_{M} is less than 100 Hz, or between 0.1 Hz and 100 Hz, preferably between 1 Hz and 50 Hz.

4. The method for cosmetic skin smoothing according to any of the preceding claims, **characterized in that** at least two different modulation frequencies are used during the treatment.

5. The method for cosmetic skin smoothing according to any of the preceding claims, **characterized in that** the energy input produced in the tissue to be treated is adjusted to less than 1 mW/l, preferably less than 100 *µ*W/l, more preferably less than 10 *µ*W/l.

6. The method for cosmetic skin smoothing according to any of the preceding claims, **characterized in that** the modulation of the field strength of the quasi-static magnetic field and/or of the frequency of the alternating magnetic field causes a fast adiabatic passage in the tissue to be treated.

7. The method for cosmetic skin smoothing according to any of the preceding claims, **characterized in that** the modulation of the field strength of the quasi-static magnetic field is at least 5 % of the amplitude of the minimum field strength of the quasi-static magnetic field.

8. The method for cosmetic skin smoothing according to any of the preceding claims, **characterized in that** the modulation of the frequency of the alternating magnetic field is at least 5 % of the minimum frequency of the alternating magnetic field.

9. The method for cosmetic skin smoothing according to any of the preceding claims, **characterized in that** the modulation of the field strength of the quasi-static magnetic field is at least twice the magnetic field strength of the terrestrial magnetic field.

10. The method for cosmetic skin smoothing according to any of the preceding claims, **characterized in that** the treatment time is between 1 and 150 min.

11. The method for cosmetic skin smoothing according to any of the preceding claims, wherein the field strength of the quasi-static magnetic field is adjusted to less than 50 Gauss.

12. The method for cosmetic skin smoothing according to any of the preceding claims, wherein the frequency of the alternating magnetic field is less than 100 kHz.

13. The method for cosmetic skin smoothing according to any of the preceding claims, wherein the modulation of the field strength of the quasi-static magnetic field and/or of the frequency of the alternating magnetic field causes a fast adiabatic passage in the tissue to be treated, wherein the modulation frequency is chosen such that magnetization is at least 60 % of the maximum magnetization in the tissue to be treated.

## Revendications

1. Procédé pour le lissage cutané cosmétique, comportant les étapes suivantes :
- production d'un champ magnétique quasi-statique dans la zone de la peau à lisser,
- production simultanée d'un champ alternatif magnétique dans la zone de la peau à lisser, lequel est au moins par zones sensiblement perpendiculaire au champ magnétique quasi-statique,
- modulation de l'intensité du champ magnétique quasi-statique et/ou de la fréquence du champ alternatif magnétique,
- une crème étant appliquée pendant la production des champs magnétiques, laquelle contient les constituants suivants :
- eau,
- acide hyaluronique,
- ubiquinone,
- collagène,
- vitamine E.

2. Procédé pour le lissage cutané cosmétique selon la revendication précédente, le procédé étant **caractérisé en ce qu'**il est appliqué en cas de cellulite.

3. Procédé pour le lissage cutané cosmétique selon la revendication précédente, **caractérisé en ce que** l'intensité du champ magnétique quasi-statique et/ou la fréquence du champ alternatif magnétique sont modulées par une fréquence de modulation f_{M}, ladite fréquence de modulation f_{M} étant inférieure à 100 Hz ou se situant entre 0,1 Hz et 100 Hz, de préférence entre 1 Hz et 50 Hz.

4. Procédé pour le lissage cutané cosmétique selon l'une quelconque des revendications précédentes, **caractérisé en ce qu'**au moins deux fréquences de modulation différentes sont utilisées pendant le traitement.

5. Procédé pour le lissage cutané cosmétique selon l'une quelconque des revendications précédentes, **caractérisé en ce que** l'apport d'énergie générée dans le tissu à traiter est réglée à moins de 1 mW/l, de préférence à moins de 100 *µ*W/l, encore mieux à moins de 10 *µ*W/l.

6. Procédé pour le lissage cutané cosmétique selon l'une quelconque des revendications précédentes, **caractérisé en ce qu'**un passage adiabatique rapide dans le tissu à traiter se produit sous l'effet de la modulation de l'intensité du champ magnétique quasi-statique et/ou de la fréquence du champ alternatif magnétique.

7. Procédé pour le lissage cutané cosmétique selon l'une quelconque des revendications précédentes, **caractérisé en ce que** la modulation de l'intensité du champ magnétique quasi-statique correspond au moins à 5 % de l'amplitude de l'intensité minimum du champ magnétique quasi-statique.

8. Procédé pour le lissage cutané cosmétique selon l'une quelconque des revendications précédentes, **caractérisé en ce que** la modulation de fréquence du champ alternatif magnétique correspond au moins à 5 % de la fréquence minimum du champ alternatif magnétique.

9. Procédé pour le lissage cutané cosmétique selon l'une quelconque des revendications précédentes, **caractérisé en ce que** la modulation de l'intensité du champ magnétique quasi-statique correspond au moins au double de l'intensité du champ magnétique terrestre.

10. Procédé pour le lissage cutané cosmétique selon l'une quelconque des revendications précédentes, **caractérisé en ce que** le temps de traitement se situe entre 1 et 150 min.

11. Procédé pour le lissage cutané cosmétique selon l'une quelconque des revendications précédentes, dans lequel l'intensité du champ magnétique quasi-statique est réglée à moins de 50 Gauss.

12. Procédé pour le lissage cutané cosmétique selon l'une quelconque des revendications précédentes, dans lequel la fréquence du champ alternatif magnétique mesure moins de 100 kHz.

13. Procédé pour le lissage cutané cosmétique selon l'une quelconque des revendications précédentes, dans lequel un passage adiabatique rapide dans le tissu à traiter se produit sous l'effet de la modulation de l'intensité du champ magnétique quasi-statique et/ou de la fréquence du champ alternatif magnétique, la fréquence de modulation étant choisie de telle sorte que la magnétisation correspond au moins à 60 % de la magnétisation maximale dans le tissu à traiter.
